# EUROPEAN PATENT APPLICATION

(11) **EP 1 857 117 A1**
(43) Date of publication of application: **21.11.2007**
(21) Application number: 06716849.2
(22) Date of filing: 23.01.2006
(51) Int. Cl.: A61K 39/395, C07K 16/28

(54) **AGENT FOR STIMULATING THE PRODUCTION OF A BLOOD COAGULATION FACTOR VIII**

(30) Priority: 25.01.2005 RU 2005102119
(71) Applicant: Bizyaev, Alexey Vyacheslavovich, Moskovskaya obl. 143300 (RU)
(72) Inventor: RUGAL, Victor Ivanovich, St.Petersburg, 192281 (RU); SELIVANOV, Eugenij Alexeevich, St.Petersburg, 193123 (RU); GONCHARE, Vladimir Alexandrovich, St.Petersburg, 194156 (RU); CRAVETS, Vasily Nikolaevich, St.Petersburg, 196240 (RU)
(74) Representative: Kador & Partner
(86) International application number: PCT/RU2006/000024
(87) International publication number: WO 2006/080867

(57) **Abstract**

The inventive agent comprises polyclonal immunoglobulins of IgG class whose molecular mass is equal to 150 kD and which are obtainable in the form of a humoral immune response to an endothelial and mesenchymal stem cells complex.

## Description

### Pertinent art

The present invention refers to medicine, namely to the medical agents which contain antibodies, namely immunoglobulins. It may be used to treat hemophilia A resulting from insufficient generation or lack of the factor VIII in a patient's organism.

Hemophilia A is a hereditary disease associated with deficiency or more rarely genetical molecular anomaly of the blood clotting factor VIII. On medical evidence 2003 [Van Dame et al Haemophilia, Nº 1, p. 94-103], it happens to about one person per 5 000 males and is characterized with spontaneous hemorrhages into soft tissues and joints that bring to serious hemorrhagic complications such as hemarthrosis, hemomiasites etc.

Hemophilia is accompanied with low immunity to virus and bacterial infections. A gravity of complications is strongly dependent upon a level of the factor VIII in a patient's plasma. A healthy person has a concentration of the factor VIII in his blood plasma of 150 ng/ml. There are three degrees of gravity of hemophilia A depending upon a concentration of the factor VIII: a mild contains 5-25% of normal concentration of the factor VIII; moderate - 1-4% and severe hemophilia - less than 1% of normal concentration of the factor VIII.

### Antecedent technical level

It is known [Nilsson I.N. Hemophilia, St.Petersburg, 1999, p.101] that a low generation or lack of the factor VIII of hemophiliacs A is associated with various causes one of which is an intense functioning of genes-suppressors of generation of the factor VIII; besides, the low generation of the factor VIII may result from a poor formation of the factor VIII producing cells by human organism. The purpose of appropriate therapy of hemophilia A is to inactivate suppressors of generation of the factor VIII, to stimulate functioning of progenitor cells of endotheliocytes and to enhance a generation of the factor VIII. However, up to the present moment there have been no medicines that can affect the above-mentioned processes.

The modem treatment of patients who suffer from hemophilia A consists in injection of fresh frozen plasma of donor's blood, cryoprecipitate and concentrates of the blood clotting factor VIII [Yakunina L.N. and others., The modem principles of children treatment who suffer from hemophilia. Aspects of haematology/oncology and immunopathology in pediatrics. 2004, v.3, Nº 2 p. 1-4]. The said treatment does not lead to recovery and may provoke a number of complications associated with infusion therapy, primarily, various forms of hepatitis. However, the main disadvantage of replacement therapy is a suppression of the own factor VIII generation with the result that a patient needs continuous regular injections of mentioned medicines.

### Disclosure of invention

The invention is based on obtaining a medicine that stimulates a generation of the blood clotting factor VIII of the patient who suffers from hemophilia A.

According to the invention, it is proposed an agent to stimulate a generation of the blood clotting factor VIII that contains polyclonal IgG-immunoglobulins of molecular weight 150 kD obtained as an humoral immune response to a complex of antigens of endothelial and mesenchymal stem cells.

The claimed agent may be obtained as a xenogenic humoral immune response to a complex of antigens of endothelial and mesenchymal stem cells.

The IgG-class and IgG1-IgG4-underclass immunoglobulins are of identical constant regions with constant sequence of amino acids and diverse variable regions; a distinction in variable regions brings to distinction of the main effector characteristics. Being generated as a result of humoral response to endothelial and mesenchymal stem cells, they stimulate functional activity of endothelial cells, regulate progression of endotheliocyte progenitor cells and activate mesenchymal stem cells.

### The claimed agent may be obtained the following way.

To obtain the antigen, a trepanobioptate of donor bone marrow containing endothelial, endosteal and periosteal mesenchymal cells was washed of blood corpuscles with a physiological solution, mechanically masticated to homogeneous state and weighed in the physiological solution.

The homogeneous suspended material was centrifugated at a speed of 1500 r/min. A supernatant fluid was being frozen trice and defrosted after which masticated again and centrifugated at a speed of 3000 r/min. A protein content in the supernatant fluid was appreciated with a biuretic method and brought to a concentration of 2,2 mg/ml by diluting with the physiological solution.

The antigen obtained this way was injected into an animal 2,2 mg/ml counting on 1 kg of an immunized object. As the immunized object may be taken any biological object capable to give humoral immune response to foreign protein introduction, for example, domestic homed cattle. The first antigen injection was carried out in an entire Freund's adjuvant or another immune response stimulator. Next injections were being held at 2-7 days intervals so that the whole immunization duration take 20-50 days. The immunized object blood is taken into germ-proof utensils without preservatives and anticoagulants. The blood sampling rate of an animal does not exceed 10 ml blood per 1 kg body weight.

After the blood corpuscles sedimentation and clot formation, the supernatant fluid (serum) was put into another germ-proof bottle. Immunoglobulin fractions were separated out of the supernatant fluid by desalting, deposits were separated, dissolved in distilled water and divided into fractions chromatographically. The fraction corresponding to IgG-class was washed away with a special column effluent and cleaned up with a dialysis through the permselective membrane. A purified solution was sterilized by filtration, divided into doses and desiccated in vacuum or inert gas ambient until residual moisture 2-5%. The agent was being kept at a temperature of 4°C in dark place not more than 24 months.

Being a source of antigen material of human endothelial and mesenchymal stem cells, there may be used periosteal, endosteal and interstitial spaces of bone marrow obtained as trepanobioptates of the bone marrow from disease-free donors. The obtained antigen containing endothelial and mesenchymal stem cells may be used immediately after being obtained or kept frozen until application.

### Embodiment of invention.

Hereafter, the present invention is illustrated by examples of preparation and application of claimed agent in vitro and in vivo.

### Example.

A 25 kg disease-free goat was immunized. Four points of its body were injected per 55 mg antigen emulsified in the entire Freund's adjuvant. After 14 days the same amount of antigen was injected 6 times parenterally without adjuvant at 2-4 days intervals. Accumulated antigen dose was 1,54 g.

Before each injection beginning with the fourth, the antibodies (immunoglobulins) titer for endothelial and mesenchymal cells was being checked. Before the fifth injection, the antibodies titer was 1:512, before the sixth injection - 1:1024, before the blood exfusion - 1:2048. In 7 days after the last antigen injection, 250 ml blood without preservatives was taken from the goat jugular vein.

The serum was separated out of the clot and blood corpuscles by centrifuging at a speed of 3000 r/min during 30 min. Out of the serum was sedimentated a poliglobulin fraction by adding a saturated solution of ammonia sulphate. The supernatant fluid was separated by centrifuging, the deposit was dissolved in distilled water, protein concentration constituted 5 mg/ml. The solution was put into the chromatographic column and after division into fractions IgG immunoglobulins were eluated 0,025 M tris-HCE pH 7,8-0,15 M tris - HCE with pH 7,8 buffer. The solution was dialyzed through the permselective membrane against distilled water within 2 days. After the dyalysis, the immunoglobulins solution was sterilized by filtering through Millipore's filters 0,22 mkm. The liquid sterile solution was put into ampullas per 1 ml. The protein content in a dose was 5 mg. Desiccation by lyophilizing was held in a regime of immunoglobulins lyophilization (from - 60°C till 37°C). The ampullas were hermetically sealed up. The agent was being kept till application at a temperature of 4°C.

The antigen titer in the prepared agent constituted 1:1200 to endothelial and mesenchymal cells antigen. A residual moisture 3% mass. The lyophilized agent represent a white porous hygroscopic mass completely dissoluble in water or physiological solution at indoor temperature within 60 sec. The ampullas content dissolves in 1 ml water, the solution is transparent and slightly opalescent.

The agent was under investigation for sterility, toxicity and apyrogenicity in accordance with regulated techniques. Specific innocence was being defined by its capability to cause hemagglutination and thromboagglutination in respective tests and lymphotoxicity in microlymphocytotoxicity by Terasaky as well [Ketlinskii S.A., Kalinina N.M. Immunology for doctor. Saint-Petersburg, 1998, 156 p], while the claimed agent concentrates action on blood corpuscle.

The agent did not possess toxic property in delutions under test. Morphological investigations under light and electronic microscopy did not reveal toxic characteristics - vacuolization and structural rupture of cytoplasm, its colouring by standard dyes, - cells organelle integrity not defected.

The claimed agent was tested on patients who suffered from severe hemophilia A. For these tests there were chosen a 14 years old patient G., 22 years old patient T. and 38 years old patient I. All of them were normally getting injections of the factor VIII by 500-600 international units and cryoprecipitate by 8-10 doses intravenously once per 2-3 days.

Using the claimed agent, the treatment took 3 days with 3 intravenous injections by dose of 0,05-0,1 mgm of the medicine per kilogram of the patient's weight. The observation took 35 days.

**The results of the research are represented in the following table.**

**Table**

| Level of the factor VIII in a blood plasma of hemophilia patient (% of normal concentration) | | | | | | |
|---|---|---|---|---|---|---|
| Patient | Age | Initial level of factor VIII | In 24 hours | In a week | In 35 days | Number of the factor VIII transfusions during the observation time |
| G. | 14 years | 0,46 | 0,40 | 1,00 | 1,20 | 1 |
| T. | 22 years | 0,42 | 0,60 | 0,63 | 0,79 | 3 |
| I. | 38 years | 1,10 | 0,80 | 1,30 | 1,10 | 2 |

As follows from the table above, the concentration of factor VIII in patient G. and patient Ts. plasma increased (after 3 injections only). As for the patient I., the factor VIII concentration did not change, however improvement of the general state of health required only 2 factor VIII transfusions within 35 days.

During the observation time a number of exogenic factor VIII transfusions was reduced to unitary per month. There were not registered contagious, catarrhal and herpetic diseases even though winter time. But the quality of life was subjectively improved, that is, no longer spontaneous hemorrhages into joints, more freedom of movements with hands and legs, better sleeping, appetite and mood of the patients.

### Feasibility.

The claimed agent may be used to treat hemophilia A and produced by biotechnological branch of the pharmaceutical industry.

## Claims

1. Agent to stimulate a generation of the blood clotting factor VIII containing polyclonal IgG-immunoglobulins of molecular weight 150kD obtained as an humoral immune response to a complex of antigens of endothelial and mesenchymal stem cells.

2. Agent to stimulate a generation of the blood clotting factor VIII according to item 1 differs because it contains xenogenic polyclonal IgG-immunoglobulins to a complex of antigens of endothelial and mesenchymal stem cells.
